# EUROPEAN PATENT SPECIFICATION

(11) **EP 2 193 116 B1**
(45) Date of publication and mention of the grant of the patent: **08.08.2012**
(21) Application number: 08803807.0
(22) Date of filing: 08.09.2008
(51) Int. Cl.: C07C 227/28, C07C 229/48

(54) **PROCESS FROM SHIKIMIC ACID TO OSELTAMIVIR PHOSPHATE**
VERFAHREN ZUR UMWANDLUNG VON SHIKIMINSÄURE IN OSELTAMIVIRPHOSPHAT
PROCÉDÉ DE L'ACIDE SHIKIMIQUE AU PHOSPHATE D'OSELTAVIMIVIR

(30) Priority: 18.09.2007 EP 07116617
(43) Date of publication of application: 09.06.2010
(73) Proprietor: F. Hoffmann-La Roche AG, 4070 Basel (CH)
(72) Inventor: TRUSSARDI, René, CH-4127 Birsfelden (CH)
(74) Representative: Zhuang Plodeck, Jianping
(86) International application number: PCT/EP2008/061838
(87) International publication number: WO 2009/037137

(56) References cited:
- EP-A- 1 127 872
- ABRECHT ET AL: "The Synthetic development of the Anti-Influenza Neuraminidase Inhibitor Oseltamivir Phosphate (Tamiflu): A challenge for Synthesis and Process Research" CHIMIA, vol. 58, no. 9, 2004, pages 621-629, XP002512247
- ROHLOFF J C ET AL: "Practical Total Synthesis of the Anti-Influenza Drug GS-4104" JOURNAL OF ORGANIC CHEMISTRY, AMERICAN CHEMICAL SOCIETY, EASTON.; US, vol. 63, no. 13, 1 January 1998 (1998-01-01), pages 4545-4550, XP002168596 ISSN: 0022-3263 cited in the application
- KIM ET AL.: "Influenza Neuraminidase Inhibitors Possessing a Novel Hydrophobic Interaction in the Enzyme Active Site: Design, Synthesis, and Structural Analysis of Carbocyclic Sialic Acid Analogues with Potent Anti-Inlfuenza Activity" J. AM. CHEM. SOC., vol. 119, 1997, pages 681-690, XP002512462

## Description

The present invention relates to a process for the preparation of a 4,5-diamino shikimic acid derivative of formula 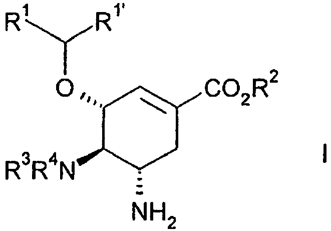 and pharmaceutically acceptable addition salts thereof
wherein
R¹, R^{1'} are independent of each other H or alkyl,
R² is an alkyl and
R³, R⁴ are independent of each other H or a C₁₋₆-alkanoyl, with the proviso that not both R³ and R⁴ are H.

4,5-diamino shikimic acid derivatives of formula I, especially the (3R,4R,5S)-5-amino-4-acetylamino-3-(1-ethyl-propoxy)-cyclohex-1-ene-carboxylic acid ethyl ester and its pharmaceutically acceptable additional salts are potent inhibitors of viral neuraminidase (J. C. Rohloff et al., J. Org. Chem. 63, 1998, 4545-4550; WO 98/07685).

The problem at the root of the present invention is to provide a new process for preparing 4,5-diamino shikimic acid derivatives in good quality and yield from a easily obtainable starting material, shikimic acid. Shikimic acid can be easily obtained from biotech processes, e.g. genetic engineering, fermentation (Sunil S. Chandran, Jian Yi, K.M. Draths, Ralph von Daeniken, Wolfgang Weber, and J.W. Frost, Biotechnologie Progress, Vol. 19, No. 3, 2003, 808-814).

The problem is solved, according to the present invention, by a process for preparing the compounds of formula I as show in scheme 1:

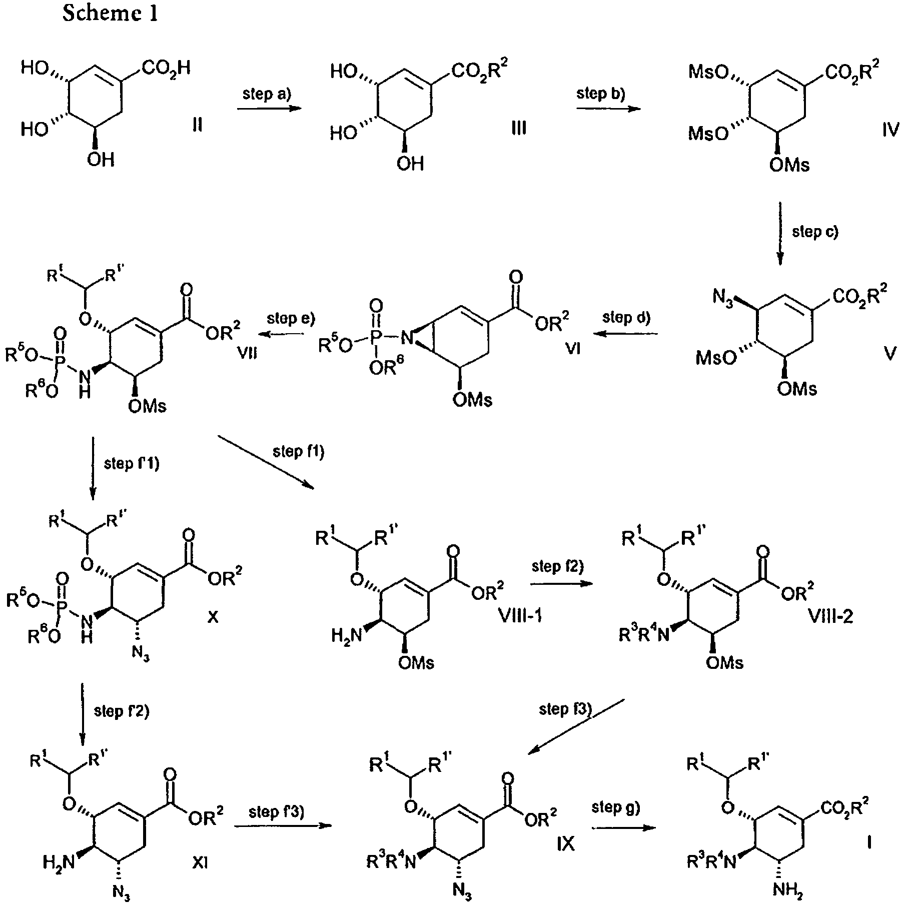

This new process has the advantage that it comprises less steps to reach 4,5-diamino shikimic acid derivatives of formula I comparing with the process known from the art (J.C.Rohloff et al., J.Org.Chem. 63, 1998, 4545-4550; WO 98/07685 and Abrecht et al: "The synthetic development of the anti-influenza neuraminidase inhibitor oseltamivir phosphate (Tamiflu): a challenge for synthesis and process research" CHIMIA Vol. 58, no. 9, 2004, pages 621-629). In addition the combination of process steps without the isolation and purification of the corresponding intermediates described in Scheme 1 allows to further enhance the efficiency and overall yield of the process

The new process characterized by
step a)
   esterification of shikimic acid of formula 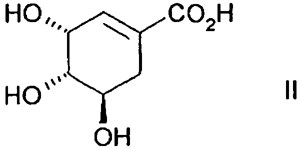 with R¹OH to form a compound of formula 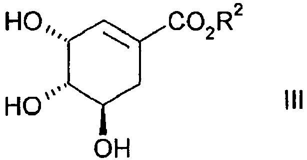 wherein R² is as defined above;
step b)
   formulation of trimesylate of formula IV by reacting compound of formula III with methanesulfonyl chloride 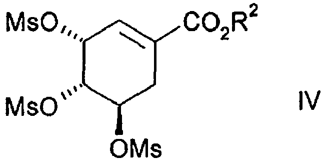 wherein R² is as defined above,
step c)
   Regioselective S_{N}2-substitution of the 3-mesylate-group of the trimesylate IV by an azide reagent to form an azide of formula 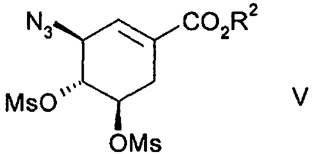 wherein R² is as defined above,
step d)
   Reduction of the azide of formula V with trialkylphosphite to form aziridine of formula 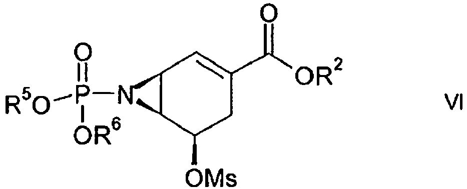 wherein R² is as defined above, R⁵ and R⁶, independently of each other are C₁₋₆ alkyl,
step e)
   opening of the aziridine of formula VI to form compound of formula 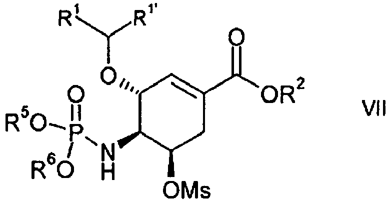 wherein R¹, R^{1'}, R², R⁵ and R⁶ are as defined above,
step f)
   transformation of phosphoramide of formula VII into compound of formula 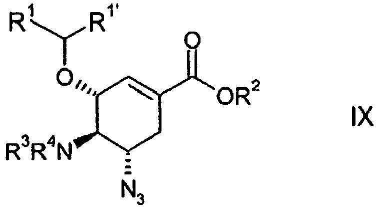 wherein R¹, R^{1'}, R², R³ and R⁴ are as defined above,
step g)
   reduction of azide of formula IX to form compound of formula I and if necessary, formation of a pharmaceutically acceptable addition salt.

There are two alternative ways for perform step f)
1. step f) comprises the steps of f1), f2) and f3:
   step f1)
      hydrolysis of phosphoramide of formula VII to form compound of formula 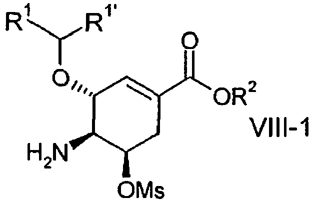 wherein R¹, R^{1'} and R² are as defined above,
   step f2)
      acylation of compound of formula VIII-1 for form compound of formula 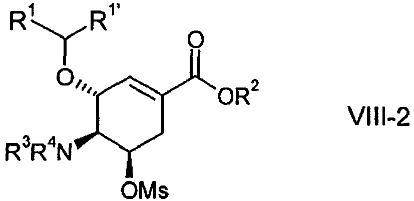 wherein R¹, R^{1'}, R², R³ and R⁴ are as defined above,
   step f3)
      substitution of 5-mesylate-group of compound of formula VIII-2 to an azido to form the compound of formula 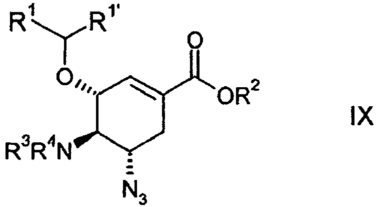 wherein R¹, R^{1'}, R², R³ and R⁴ are as defined above,
   or alternatively
2.1. step f) comprises the steps of f1), f2) and f3:
   step f'1)
      substitution of 5-mesylate-group of compound of formula VII to form the compound of formula 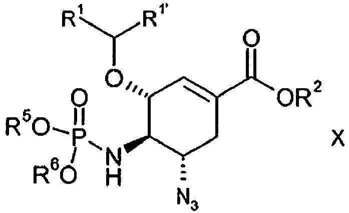 wherein R¹, R^{1'}, R², R³ and R⁴ are as defined above,
   step f2)
      hydrolysis of compound of formula X to form compound of formula 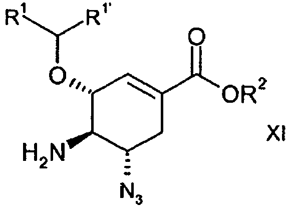 wherein R¹, R^{1'} and R² are as defined above,
   step f3)
      acylation of compound of formula XI to form compound of formula 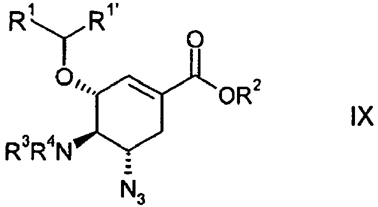 wherein R¹, R^{1'}, R², R³ and R⁴ are as defined above,

The term alkyl has the meaning of a straight chained or branched alkyl group of 1 to 12 C-atoms, expediently of 1 to 6 C-atoms. Examples of such alkyl groups are methyl, ethyl, n-propyl, i-propyl, n-butyl, i-butyl, tert. butyl, pentyl and its isomers, hexyl and its isomers, heptyl and its isomers, octyl and its isomers, nonyl and its isomers, decyl and its isomers, undecyl and its isomers and dodecyl and its isomers.

This alkyl group can be substituted with one or more substituents as defined in e.g. WO 98/07685. Suitable substituents are C₁₋₆-alkyl (as defined above), C₁₋₆-alkenyl, C₃₋₆-cycloalkyl, hydroxy, C₁₋₆- alkoxy, C₁₋₆-alkoxycarbonyl, F, Cl, Br and I.

The term alkyl in R¹, R^{1'} has the meaning of a straight chained or branched alkyl group of 1 to 12 C-atoms, expediently of 1 to 6 C-atoms.

Preferred meaning for R¹ is ethyl, for R^{1'} is ethyl.

R² is a straight chained or branched alkyl group of 1 to 12 C-atoms, expediently of 1 to 6 C-atoms as exemplified above.

Preferred meaning for R² is ethyl.

R³ and R⁴ have the meaning of alkanoyl groups, more preferably C₁₋₆-alkanoyl such as hexanoyl, pentanoyl, butanoyl (butyryl), propanoyl (propionyl), ethanoyl (acetyl) and methanoyl (formyl).

Preferred meaning for R³ is acetyl and for R⁴ is H.

R⁵ and R⁶ are straight chained or branched alkyl group of 1 to 12 C-atoms, expediently of 1 to 6 C-atoms as exemplified above.

Preferred meaning for R⁵ and R⁶ are are ethyl.

The term "pharmaceutically acceptable acid addition salts" embraces salts with inorganic and organic acids, such as hydrochloric acid, hydrobromic acid, nitric acid, sulfuric acid, phosphoric acid, citric acid, formic acid, fumaric acid, maleic acid, acetic acid, succinic acid, tartaric acid, methanesulfonic acid, p-toluenesulfonic acid, benzenesulfonic acid and the like.

The salt formation is effected with methods which are known per se and which are familiar to any person skilled in the art. Not only salts with inorganic acids, but also salts with organic acids come into consideration. Hydrochlorides, hydrobromides, sulfates, nitrates, citrates, acetates, maleates, succinates, methan-sulfonates, p-toluenesulfonates and the like are examples of such salts.

Preferred pharmaceutically acceptable acid addition salt is the 1:1 salt with phosphoric acid which can be formed preferably in ethanolic solution at a temperature of -20°C to 60°C.

### Step a)

Step a) comprises esterification of (3R,4S,5R)-3,4,5-trihydroxy-cyclohex-1-enecarboxylic acid of formula II with an alcohol of formula R²OH.

Typically, the reaction is performed in an alcohol, preferably ethanol in the presence of a strong acid, such as hydrogen chloride in ethanol, sulfuric acid or in situ preparation of hydrogen chloride with thionyl chloride in ethanol , preferably thionyl chloride in ethanol.

The reaction temperature mainly depends on the alcohol used, as a rule lies in the range of 60°C to 150°C, preferably 70°C to 100°C.

The reaction is as a rule finished after 1 to 5 hours, preferably 2 to 3 hours.

### Step b)

Step b) comprises formulation of trimesylate of formula IV by reacting compound of formula III with methanesulfonyl chloride.

Typically the reaction is performed in a suspension of compound of formula III, an aprotic organic solvent, preferably ethylacetat, and methanesulfony chloride with a tertiary organic base, preferably triethylamine.

The reaction temperature is typically in the range of -20° C to 50° C, preferably 0° C to 5°C.

### Step c)

Step c) comprises regioselective S_{N}2-substitution of the 3-mesylate-group of the trimesylate IV by an azide reagent to form an azide of formula V.

The reaction of step c) is typically performed in an inert organic solvent, such as dimethyl sulfoxide or with a water organic solvent mixture, preferably ethylacetate and with a phase transfer catalyst, such as tetrabutylammonium hydrogen sulfate.

An azide reagent, preferably sodium azide is added to the reaction mixture at room temperature.

The reaction is as a rule finished after 1 to 30 hours.

### Step d)

Step d) comprises reduction of the azide of formula V with trialkylphosphite to form aziridine of formula VI.

The reaction of step d) is typically performed in an organic solvent, such as toluene in an inert gas atmosphere. Trialkylphosphite, such as triethyphosphite is added at room temperature.

The reaction is as a rule finished after 1 to 5 hours, preferably 2 to 3 hours.

The reaction temperature mainly depends on the organic solvent used, as a rule lies in the range of 50°C to 150°C, preferably 100°C to 120°C.

### Step e)

Step e) comprises the opening of the aziridine of formula VI to form compound of formula VII.

Typically the reaction is performed in a suspension of compound of formula VI, an alcohol, such as 3-pentanol and boron trifluoride ethyl etherate in an inert gas atmosphere.

The reaction temperature is typically in the range of -20°C to 100°C, preferably 0°C to 5°C.

The reaction is as a rule finished after 10 to 20 hours, preferably 16 hours.

### Step f)

Step f) can be performed in two alternative ways:
(1) step f) comprises steps f1), f2) and f3.
   Step f1) comprises substitution of mesylate-group of compound of formula VII to form the compound of formula VIII-1.
      Typically the reaction is performed in a suspension of compound of formula VII, an alcohol, such as ethanol and a strong acid, such as hydrochloric acid, hydrobromic acid, sulfuric acid, phosphoric acid, methanesulfonic acid and the like, preferably sulfuric acid in an inert gas atmosphere.
      The reaction temperature mainly depends on the alkohol used, as a rule lies in the range of 50°C to 150°C, preferably 80°C to 120°C.
      The reaction is as a rule finished after 10 to 20 hours, preferably 16 hours.
   Step f2) comprises acylation of compound of formula VIII-1 for form compound of formula VIII-2.
      Acylation can be effected under basic conditions by using acylating agents known to the skilled in the art. Acylating agent can be an acyl halide, a carboxylic acid ester or a carboxylic acid anhydride. Suitable acylating agent preferably is an acetylating agent such as acetylchloride or acetic anhydride.Suitable organic tertiary base such as triethylamine or an aqueous solution with an anorganic base such sodium hydrogen carbonate, potassium carbonate, potassium phosphate in water preferably sodium hydrogen carbonate in water.
      Preferably the acylation takes place under basic conditions using a mixture of 0.5 to 2.0 equivalents of acetic anhydride in ethyl acetate.
      The temperature is as a rule chosen in the range of -20°C to 100°C.
   Step f3) comprises substitution of mesylate-group of compound of formula VIII to an azido to form the compound of formula IX.
      The reaction of step f3) is typically performed in an inert organic solvent, such as dimethyl sulfoxide or a solvent mixture with dimethyl sulfoxide and ethanol.
      An azide reagent, such as sodium azide is added to the reaction mixture at 20°C to 120°C, preferably at 80°C to 100°C
      The reaction is as a rule finished after 10 to 30 hours, preferably 16 hours.
(2) step f) comprises steps f1), f2) and f3).
   Step f1) comprises substitution of mesylate-group of compound of formula VII to form the compound of formula X.
      The reaction of step f1) is typically performed in an inert organic solvent, such as dimethyl sulfoxide or an solvent mixture with dimethyl sulfoxide and ethanol.
      An azide reagent, such as sodium azide is added to the reaction mixture at 20°C to 120°C, preferably at 80°C to 100°C.
      The reaction is as a rule finished after 10 to 30 hours, preferably 20 hours.
   Step f2) comprises hydrolysis of compound of formula X to form compound of formula XI.
      Typically the reaction is performed in a suspension of compound of formula X, an alcohol, such as ethanol and a strong acid, such as hydrochloric acid, hydrobromic acid, sulfuric acid, phosphoric acid, methanesulfonic acid and the like, preferably sulfuric acid in an inert gas atmosphere.
      The reaction temperature mainly depends on the alcohol used, as a rule lies in the range of 20°C to 150°C, preferably 80°C to 120°C.
      The reaction is as a rule finished after 10 to 20 hours, preferably 8 hours at 80°C.
   Step f3) comprises acylation of compound of formula XI to form compound of formula IX.
      Acylation can be effected under basic conditions by using acylating agents known to the skilled in the art. Acylating agent can be an acyl halide, a carboxylic acid ester or a carboxylic acid anhydride. Suitable acylating agent preferably is an acetylating agent such as acetylchloride or acetic anhydride. Suitably organic tertiary base such as triethylamine or an aqueous solution with an anorganic base such as sodium hydrogen carbonate, potassium carbonate, potassium phosphate in water, preferably sodium hydrogen carbonate in water.
      Preferably the acylation takes place under basic conditions using a mixture of 0.5 to 2.0 equivalents of acetic anhydride in ethyl acetate.
      The temperature is as a rule chosen in the range of -20°C to 100°C, preferably 0 °C to 5 °C.

### Step g)

Step g) comprises reduction of azide of formula IX to form compound of formula I and if necessary, formation of a pharmaceutically acceptable addition salt.

Typically the reduction is performed in a polar protic solvent such as alcohols, preferably in aqueous ethanol or aqueous tetrahydrofuran, most preferably in aqueous ethanol.

The reaction temperature mainly depends on the phosphine used but as a rule lies in the range of -20°C to 30°C, preferably between 0 and 25°C.

It can be favorable to perform the reaction at two temperature levels, thereby having the lower temperature range given above for the addition of the phosphine and thereafter having a slightly higher temperature of up to room temperature to bring the reaction to completion.

As a specific embodiment of the present invention it was found that catalytic amounts of an acid present during the conversion suppresses the ester hydrolysis which otherwise takes place to a small extent of some percent and thereby leads to an undesirable impurity.

Suitable acid is a carboxylic acid, expediently acetic acid. The acetic acid is usually added in the form of glacial acetic acid in catalytic quantities of 0.5 mol % to 3.0 mol % relating to the 4-acetylamino-5-azido-shikimic acid derivative of formula (IX).

The process is as a rule finished after 3 h to 6 h.

Thereafter work up of the reaction mixture can happen by applying methods known to the skilled in the art. Expediently the reaction mixture is, preferably after stabilization with ≤ 5 mol% acetic acid, concentrated in vacuo.

Though the 4,5-diamino shikimic acid derivative can be isolated e.g. by evaporation and crystallization, it is preferably kept in e.g. an ethanolic solution and then further transformed into the pharmaceutically acceptable addition salt following the methods described in J.C.Rohloff et al., J.Org.Chem. 63, 1998,4545-4550; WO 98/07685).

The salt formation is effected with methods which are known per se and which are familiar to any person skilled in the art. Not only salts with inorganic acids, but also salts with organic acids come into consideration. Hydrochlorides, hydrobromides, sulphates, nitrates, citrates, acetates, maleates, succinates, methan-sulphonates, p-toluenesulphonates and the like are examples of such salts.

Preferred pharmaceutically acceptable acid addition salt is the 1:1 salt with phosphoric acid which can be formed preferably in ethanolic solution at a temperature of -20°C to 60°C.

The following examples shall illustrate the invention in more detail without limiting it.

### Example 1

### (a) Preparation of (3R,4S,5R)-3,4,5,-Trihydroxy-cyclohex-1-enecarboxylic acid ethyl ester (iii)

In a 250 ml four necked round bottom flask equipped with a reflux condenser, magnetic stirrer and a inert gas supply 19.90 g (3R,4S,5R)-3,4,5-Trihydroxy-cyclohex-1-enecarboxylic acid (ii) were suspended in 80 ml ethanol, 4.16 ml thionyl chloride were added in a period of 10 min. The reaction mixture was heated to reflux for 2 hours, the brown solution was cooled to room temperature and evaporated at 40°C/200-10 mbar to yield the crude product 24.39 g as dark brown oil which crystallized over night. The crude product was dissolved in 100 ml ethyl acetate and evaporated at 40°C/200-10 mbar to yield the product 22.56 g as a brown residue.

IR (ATR) 3350, 2982, 1715, 1252, 1097 cm-1; MS (turbo spray) MNH₄⁺ 220.1

### (b) Preparation of (3R,4S,SR)-3,4,5-Tris-methanesulfonyloxy-cyclohex-1-enecarboxylic acid ethyl ester (iv)

In a 1000 ml two necked round bottom flask equipped with, a thermometer, a mechanic stirrer and an inert gas supply 20.22 g (3R,4S,5R)-3,4,5,-Trihydroxy-cyclohex-1-enecarboxylic acid ethyl ester (iii) was suspended in 600 ml ethyl acetate, cooled to 0-5°C, 24.0 ml methanesulfonyl chloride were added. To the dark brown solution 45.0 ml triethylamine were added in a period of 46 min, were by the temperature was kept in the range of 0-5°C. The brown suspension was stirred for 30 min at 0-5°C, filtered over a pre cooled (0-5°C) glass frit filter, the filter cake was washed with total 200 ml ethyl acetate, the combined filtrates were extracted with 400 ml 0.5M H₂SO₄, the separated organic phase was dried with 100 g anhydrous sodium sulfate, filtered, washed with 200 ml ethyl acetate and evaporated in a rotary evaporator at 40°C/200-10 mbar to yield the product 42.24 g as a brown resin

IR (ATR) 2943, 1715, 1332, 1251, 1170 cm⁻¹; MS (ion spray): 454.2 M+NH₄⁺,

### (c) Preparation of (3R,4S,SR)-3-Azido-4,5-bis-methanesulfonyloxy-cyclohex-1-enecarboxylic acid ethyl ester (v)

In a 5 ml round bottom flask equipped with a magnetic stirrer and an inert gas supply, 436.3 mg (3R,4S,5R)-3,4,5-Tris-methanesulfonyloxy-cyclohex-1-enecarboxylic acid ethyl ester (iv) were dissolved in 0.83 ml dimethyl sulfoxide. To the clear colorless solution 71.5 mg sodium azide were added at r.t., the reaction mixture was stirred for 2.5 hour, then 13.7 mg sodium azide were added at r.t. and stirred for another 19 hour at r.t. The reaction mixture was diluted with 5 ml ethyl acetate and extracted three times with a 1M solution of NaHCO₃. The organic phase was separated dried with anhydrous Na₂SO₄, filtered and evaporated at 20°C/140-10 mbar to obtain 0.33 g of the crude product as light yellow oil. Purification of the crude product was obtained via a silica column chromatography using a 1/2 mixture of ethyl acetate and n-hexane. The combined fractions were evaporated and dried on a rotary evaporator to obtain 0.27 g (3R,4S,5R)-3-Azido-4,5-bis-methanesulfonyloxy-cyclohex-1-enecarboxylic acid ethyl ester as a colorless oil

IR (ATR) 2942, 2105, 1713, 1660, 1350, 1171 cm-1. MS (turbo spray) M+NH₄⁺ 401.1

### (d) Preparation of (1S,5R,6S)-7-(Diethoxy-phosphoryl)-5-methanesulfonyloxy-7-aza-bicyclo[4.1.0]hept-2-ene-3-carboxylic acid ethyl ester (vi)

In a 10 ml round bottom flask equipped with a magnetic stirrer, a reflux condenser and an inert gas supply, 383.4 mg (3R,4S,5R)-3-Azido-4,5-bis-methanesulfonyloxy-cyclohex-1-enecarboxylic acid ethyl ester (v) were dissolved under argon with stirring in 4.0 ml toluene. To the clear solution 191 µl triethyl phosphite were added at r.t.. After 5 minutes were nitrogen was evolved and after 30 minutes stirring at r.t. the light yellow reaction mixture was heated to reflux for 5 hours, then 191 µl triethyl phosphite was added and refluxed for another 2 hours. The reaction mixture was cooled and evaporated at 40°C/60-10 mbar to yield 470 mg of the crude product as a yellow oil. Purification of the crude product was obtained via a silica column chromatography using a 2/1 mixture of toluene and acetone. The combined fractions were evaporated and dried on a rotary evaporator 40°C/250-10 mbar to obtain 87 mg (1S,5R,6S)-7-(Diethoxy-phosphoryl)-5-methanesulfonyloxy-7-aza-bicyclo[4.1.0]hept-2-ene-3-carboxylic acid ethyl ester as a yellow oil.

IR (ATR) 2984, 1711, 1649, 1356, 1253, 1173, 1019 cm-1. MS (ion spray) MH+398.0

### (e) Preparation of (3R,4S,5R)-4-(Diethoxy-phosphorylamino)-3-(1-ethyl-propoxy)-5-methanesulfonyloxy-cyclohex-1-enecarboxylic acid ethyl ester (vii)

In a 50 ml round bottom flask equipped with a magnetic stirrer and an inert gas supply, 1.94 g (1S,5R,6S)-7-(Diethoxy-phosphoryl)-5-methanesulfonyloxy-7-aza-bicyclo[4.1.0]hept-2-ene-3-carboxylic acid ethyl ester (vi) were dissolved under argon with stirring in 20 ml 3-pentanol. The clear light yellow solution was treated at 0-5°C with 736 µl boron trifluoride ethyl etherate. The reaction mixture was stirred for 16 hours at r.t.. the reaction mixture was diluted with 50 ml ethyl acetate and extracted two times with 70 ml water. The organic phase was separated and dried with 25 g anhydrous Na₂SO₄, filtered and evaporated in a rotary evaporator at 40°C/70-10 mbar to yield 2.18 g of the crude product as a yellow oil. Purification of the crude product was obtained via a column chromatography using tert-butyl methyl ether. The combined fractions were evaporated and dried on a rotary evaporator at 40°C/250-10 mbar to obtain 1.47 g (3R,4S,5R)-4-(Diethoxy-phosphorylamino)-3-(1-ethyl-propoxy)-5-methanesulfonyloxy-cyclohex-1-enecarboxylic acid ethyl ester as a light yellow solid.

IR (ATR) 3236, 1711, 1246, 1024 cm⁻¹. MS (turbo spray) MH⁻484.2

### (f1) Preparation of (3R,4S,5R)-4-amino-3-(1-ethyl-propoxy)-5-methanesulfonyloxy-cyclohex-1-enecarboxylic acid ethyl ester (viii-1)

In a 5 ml round bottom flask equipped with a magnetic stirrer, a reflux condenser and an inert gas supply, 120 mg (3R,45,5R)-4-(Diethoxy-phosphorylamino)-3-(1-ethyl-propoxy)-5-methanesulfonyloxy-cyclohex-1-enecarboxylic acid ethyl ester (vii) was dissolved in 500 µl ethanol and treated with 120 µl sulphuric acid 96%, the reaction mixture was refluxed for 16 h, cooled to r.t. and diluted with 1.0 ml ethyl acetate and cooled to 05°C. The cooled solution was treated with 100 µl of a 28% aqueous sodium hydroxide solution and with 257 µl of a 25% aqueous ammonium hydroxide solution. The well stirred mixture was diluted with 1.5 ml water, extracted and the organic phase was separated, dried over anhydrous Na₂SO₄, filtered and evaporated at 20°C/120-10 mbar and dried for 1h at 20°C/<10 mbar to obtain 56 mg (3R,45,5R)-4-amino-3-(1-ethyl-propoxy)-5-methanesulfonyloxy-cyclohex-1-enecarboxylic acid ethyl ester as colorless oil.

IR (ATR) 1711, 1655, 1350, 1246, 1171 cm-1. MS (ion spray) MH⁺350.3

### (f2) Preparation of (3R,4S,5R)-4-Acetylamino-3-(1-ethyl-propoxy)-5-methanesulfonyloxy-cyclohex-1-enecarboxylic acid ethyl ester (viii-2)

In a 10 ml round bottom flask equipped with a magnetic stirrer and an inert gas supply, 190 mg (3R,45,5R)-4-amino-3-(1-ethyl-propoxy)-5-methanesulfonyloxy-cyclohex-1-enecarboxylic acid ethyl ester (vii-1) was dissolved in 1.6 ml ethyl acetate, 1.6 ml of a aqueous 1M sodium hydrogen carbonat solution was added, at room temperature 62 µl acetic anhydride was added, the 2-phase mixture was stirred for 1 hour at room temperature. The mixture was diluted with 2.0 ml ethyl acetate, the organic phase was separated, dried with anhydrous sodium sulfate, filtered and the filter cake was washed with total 3.0 ml ethyl acetate. The filtrate was evaporated at 40°C/200-10mbar to obtain 192 mg (3R,4S,5R)-4-Acetylamino-3-(1-ethyl-propoxy)-5-methanesulfonyloxy-cyclohex-1-enecarboxylic acid ethyl ester as off white crystals

IR (ATR) 3303, 1714, 1648, 1534, 1344, 1251, 1174, 1094, 903 cm⁻1. MS (ion spray) MH⁺392.0

### (f3) Preparation of (3R,4R,5S)-4-Acetylamino-5-azido-3-(1-ethyl-propoxy)cyclohex-1-enecarboxylic acid ethyl ester (ix)

In a 5.0 ml round bottom flask equipped with a magnetic stirrer, a reflux condenser and an inert gas supply, 340 mg (3R,4S,5R)-4-Acetylamino-3-(1-ethyl-propoxy)-5-methanesulfonyloxy-cyclohex-1-enecarboxylic acid ethyl ester (viii-2) was dissolved in 1.2 ml dimethyl sulfoxide and 1.2 ml ethanol, 113 mg sodium azide was added and the reaction mixture was stirred for 21 hours at 85-90°C. The brown reaction mixture was cooled to r.t., treated with 7.0 ml of a aqueous 1M sodium hydrogen carbonate solution and extracted with 7.0 ml tert-butyl methyl ether. The organic phase was dried with anhydrous Na₂SO₄, filtered and evaporated at 40°C/250-10mbar to obtain 280 mg of the crude product as brown crystals. 280 mg of the crude product was dissolved in 3.0 ml tert-butyl methyl ether, the brown solution was stirred 16 hours at -20°C, were by a beige suspension was formed. The suspension was filtered over a pre cooled (-20°C) glass filter frite and washed with pre cooled (-20°C) 1.5 ml tert-butyl methyl ether. The white crystals were dried at 40°C/10mbar for 2 hour to obtain 60 mg (3R,4R,5S)-4-Acetylamino-5-azido-3-(1-ethyl-propoxy)cyclohex-1-enecarboxylic acid ethyl ester as white crystals

IR (ATR) 3265, 2101, 1713, 1659, 1559, 1249, 1078 cm⁻1. MS (ion spray) MH⁺339.3

### (f'1) Preparation of (3R,4R,5S)-5-Azido-4-(diethoxy-phosphorylamino)-3-(1-ethyl-propoxy)-cyclohex-1-enecarboxylic acid ethyl ester (x)

In a 5.0 ml round bottom flask equipped with a magnetic stirrer, a reflux condenser and an inert gas supply, 104 mg of (3R,4S,5R)-4-(Diethoxy-phosphorylamino)-3-(1-ethyl-propoxy)-5-methanesulfonyloxy-cyclohex-1-enecarboxylic acid ethyl ester (vii) was dissolved in 0.50 ml ethanol and 0.50 ml dimethyl sulfoxide, 28 mg sodium azide was added and the reaction mixture was stirred at 85-90°C for 20 hours. The brown reaction mixture was cooled to room temperature diluted with 1.0 ml ethyl acetate and extracted with 1.0 ml of a aqueous 1M sodium hydrogen carbonate solution. The separated organic phase was dried with anhydrous Na₂SO₄, filtered and evaporated at 40°C/120-10mbar to obtain 40 mg of the crude product as brown crystals. 40 mg of the crude product was dissolved in 0.40 ml methylcyclohexane, stirred for 5 hours at room temperature, filtered and washed with 0.15 ml methylcyclohexane, the white crystals were dried at 40°C/10mbar for 2 hours to obtain 14 mg (3R,4R,5S)-5-Azido-4-(diethoxy-phosphorylamino)-3-(1-ethyl-propoxy)-cyclohex-1-enecarboxylic acid ethyl ester as white crystals.

IR (ATR) 3205, 2099, 1717, 1660, 1246, 1224, 1025 cm-1. MS (ion spray) M+OAc⁻491.3

### (f'2) Preparation of (3R,4R,5S)-4-Amino-5-azido-3-(1-ethyl-propoxy)-cyclohex-1-enecarboxylic acid ethyl ester (xi)

In a 5.0 ml round bottom flask equipped with a magnetic stirrer, a reflux condenser and an inert gas supply, 300 mg of (3R,4R,5S)-5-Azido-4-(diethoxy-phosphorylamino)-3-(1-ethyl-propoxy)-cyclohex-1-enecarboxylic acid ethyl ester (x) was dissolved in 1.25 ml ethanol and treated with 0.31 ml sulfuric acid 96%. The black solution was stirred for 18 hours at room temperature and 8 hours at 80°C. The black reaction mixture was cooled to room temperature treated with 8.0 ml of a mixture 65 ml ammonium hydroxide 25% in 435 ml brine, and extracted with 9.0 ml ethyl acetate. The separated organic phase was washed with 10 ml water and the water phase was washed back with 9.0 ml ethyl acetate. The comined organic phases were dried with 20 g anhydrous Na₂SO₄, filtered and evaporated at 40°C/140-10mbar to obtain 175 mg crude product as a dark brown oil. Purification of the crude product was obtained via a silica column chromatography using ethyl acetate with 1% v/v of aqueous ammonium hydroxide 25%. The combined fraction were evaporated at 40°C/140-10mbar to obtain 97 mg of (3R,4R,5S)-4-Amino-5-azido-3-(1-ethyl-propoxy)-cyclohex-1-enecarboxylic acid ethyl ester as a brown oil.

IR (ATR) 3393, 2098, 1713, 1241, 1070 cm-1. MS (ion spray) MH⁺297.2

### (f'3) Preparation of (3R,4R,5S)-4-Acetylamino-5-azido-3-(1-ethyl-propoxy)cyclohex-1-enecarboxylic acid ethyl ester (ix)

In a 5.0 ml round bottom flask equipped with a magnetic stirrer and an inert gas supply, 118.5 mg (3R,4R,5S)-4-Amino-5-azido-3-(1-ethyl-propoxy)-cyclohex-1-enecarboxylic acid ethyl ester (xi) was dissolved in 1.0 ml ethyl acetate. The brown solution was treated with 1.0 ml of a aqueous 1M sodium hydrogen carbonate solution. The mixture was treated with stirring with 45.4 µl acetic anhydride. The mixture was stirred for 1 hour at room temperature. The organic phase was separated and dried with 1g anhydrous Na₂SO₄, filtered and the filter cake was washed total with 2.0 ml ethyl acetate. The combined filtrate was evaporated at 40°C/240 to 10 mbar to obtain 139 mg crude product as beige solide. The crude solide was suspended in 1.40 ml n-hexane and 0.70 ml tert-butyl methyl ether, stirred for one hour at room temperature, cooled to 0-5°C and stirred for 2 hours at 0-5°C. The suspension was filtered and the filter cake was washed with total 0.70 ml of a precooled (0-5°C) 2/1 solvent mixture of n-hexane and tert-buthyl methyl ether. The filter cake was dried at 45°C/9mbar to obtain 94 mg (3R,4R,5S)-4-Acetylamino-5-azido-3-(1-ethyl-propoxy)cyclohex-1-enecarboxylic acid ethyl ester as white crystals.

IR (NJL) 3269, 2104, 1715, 1660, 1562, 1254 cm-1. MS (turbo spray) MH⁺339.2.

### (g) Preparation of (3R,4R,5S)-4-acetylamino-5-amino-3-(1-ethyl-propoxy)cyclohex-1-enecarboxylic acid ethyl ester (i)

50.0 g (0.147 mol) ethyl (3R, 4R, 5S)-4-acetamido-5-azido-3-(1-ethylpropoxy)-1-cyclohexene-1-carboxylate (ix) were placed in a nitrogen purged 1000 ml glass reactor fitted with a mechanical stirrer, a condenser, and a 250 ml dropping funnel. 300 ml ethanol, 50 ml water and 0.09 g acetic acid were added. To the resulting clear solution 31.4 g (0.155 mol) tributylphosphine dissolved in 150 ml ethanol were continuously added at a temperature of 5 °C (+/- 5°C) over a period of 30 - 90 min. Under slight cooling of the jacket (~ 3°C) the reaction temperature was kept at this temperature. The feeder was rinsed with 20 ml ethanol. The clear reaction mixture was stirred for additional 90 min at 5 °C (+/-5°C) under slight jacket cooling. Subsequently the temperature was raised within 30 - 60 min to 20 - 25 °C and the solution was stirred for another 3h (nitrogen evolving).

After the reaction was finished (HPLC control) 0.18 g acetic acid were added to the clear solution. Then the mixture was concentrated under reduced pressure (300 to 50 mbar) at a maximum temperature of 60 °C and a maximum jacket temperature of 75 °C near to dryness. The oily residue (80 - 100 ml) was diluted with 160 ml ethanol, the resulting solution was then again concentrated following the method as mentioned above. The oily residue was dissolved in ethanol up to a volume of 250 ml. The water content of this solution was determined by KF(Karl Fischer) titration of being less than 1.0 % wt. %. Yield: 44.4 g (97 % area by HPLC) of the title product in ethanolic solution.

IR (NJL) 3279, 1720, 1639, 1554, 1252, 1128 cm⁻1. MS (ion spray) MH⁺ 313.1, MNa⁺ 335.3

### (g1)Preparation of (3R,4R,5S)-4-acetylamino-5-amino-3-(1-ethyl-propoxy)cyclohex-1-enecarboxylic acid ethyl ester (i) phosphate (1:1)

In a dry and nitrogen purged 1000 ml glass reactor fitted with a mechanical stirrer, a condenser, and a 500 ml dropping funnel 17.0 g ortho phosphoric acid (85 % in water) were dissolved in 400 ml ethanol and the resulting clear solution was warmed to 50 - 55 °C. Subsequently the 250 ml ethanolic solution obtained from example 1 and containing 0.147 mol of ethyl (3R, 4R, 5S)-4-acetamido-5-amino-3-(1-ethylpropoxy)-1-cyclohexene-1-carboxylate were added under stirring. After fast addition (10-15 min) of two thirds (ca.160 ml) of the total volume of this solution the addition was stopped and the supersaturated clear solution was seeded with 0.2 g of previously obtained ethyl (3R, 4R, 5S)-4-acetamido-5-amino-3-(1-ethylpropoxy)-1-cyclohexene-1-carboxylate Phosphate (1:1). Immediately afterwards crystallization commenced. The resulting thick suspension was stirred for 45 - 60 min at 50 - 55 °C. Then the remaining amine solution was slowly added (45 - 60 min) to the suspension at 50 - 55 °C. The feeder was rinsed with 20 ml ethanol. Subsequently the thick suspension was continuously cooled to 12 - 20°C in about 4 h (cooling speed = 10 °C/h). To complete the crystallization stirring was continued at 12 - 20 °C for additional 2 ± 1 h. Ethyl (3R, 4R, 5S)-4-acetamido-5-amino-3-(1-ethylpropoxy)-1-cyclohexene-1-carboxylate Phosphate (1:1) was isolated by pressure filtration (0.3 bar nitrogen overpressure, Dacron^{®} filter cloth). The crystalline product was washed twice with 240 ml acetone and twice with 300 ml n-heptane at room temperature. Ethyl (3R, 4R, 5S)-4-acetamido-5-amino-3-(1-ethylpropoxy)-1-cyclohexene-1-carboxylate Phosphate (1:1) was dried in vacuo (≈20 mbar) at a maximum temperature of 50°C until constant weight.
Yield: 54 - 55 g (88-91%) of the title product in the form of colorless needles with an assay of = 99 wt. % (sum of impurities < 0.5 wt.%, single impurities ≤ 0.1 wt.%).

IR (NJL) 3352, 3164, 2923, 2854, 1724, 1663, 1551, 1463, 1263, 1132 cm-1. MS (ion spray) MH⁺ 313.1, MNa⁺ 335.3.

## Claims

1. Process for the preparation of a 4,5-diamino shikimic acid derivative of formula 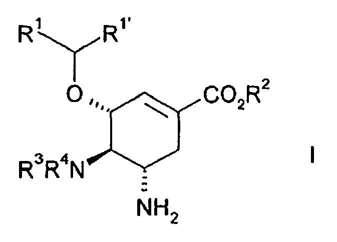 and pharmaceutically acceptable addition salts thereof,
wherein
R', R^{1'} are independent of each other H or C₁₋₆alkyl,
R² is an C₁₋₆alkyl and
R³, R⁴ are independent of each other H or an C₁₋₆-alkanoyl, with the proviso that not both R³ and R⁴ are H,
**characterized in that**
step a)
esterification of shikimic acid of formula 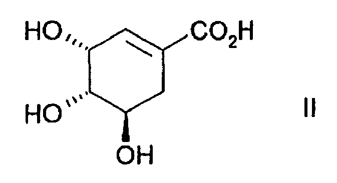 with R²OH to form a compound of formula 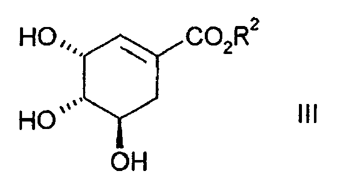 wherein R² is as defined above;
step b)
formulation of trimsylate of formula IV by reacting compound of formula III with methanesulfonyl chloride 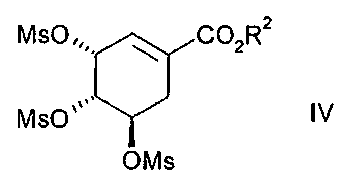 wherein R² is as defined above,
step c)
Regioselective S_{N}2-substitution of the 3-mesylate-group of the trimesylate IV by an azide reagent to form an azide of formula 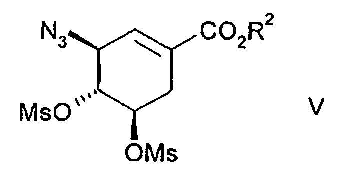 wherein R² is as defined above,
step d)
Reduction of the azide of formula V with trialkylphosphite to form aziridine of formula 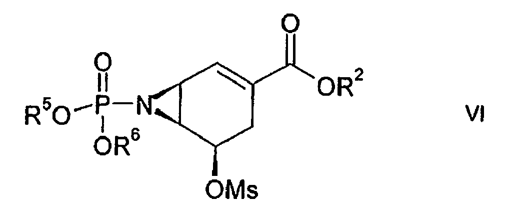 wherein R² is as defined above, R⁵ and R⁶, independently of each other are C₁₋₆alkyl,
step e)
opening of the aziridine of formula VI to form compound of formula 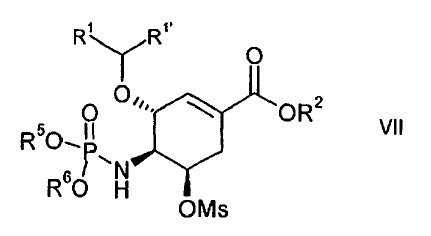 wherein R¹, R^{1'}, R², R⁵ and R⁶ are as defined above,
step f)
transformation of phosphoramide of formula VII into compound of formula 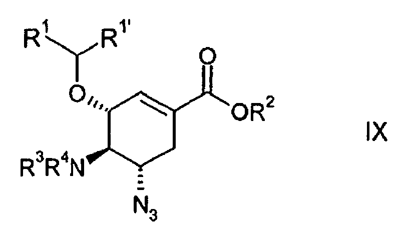 wherein R¹, R^{1'}, R², R³ and R⁴ are as defined above,
step g)
reduction of azide of formula IX to form compound of formula I and if necessary, formation of a pharmaceutically acceptable addition salt.

2. Process of claim 1, wherein step f) comprises the steps,
step f1)
hydrolysis of phosphoramide of formula VII to form compound of formula 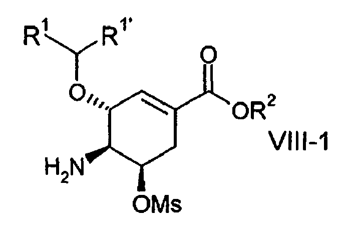 wherein R¹, R^{1'} and R² are as defined above,
step f2)
acylation of compound of formula VIII-1 for form compound of formula 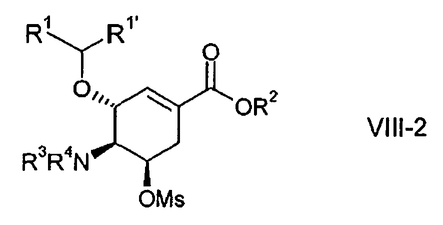 wherein R¹, R^{1'}, R², R³ and R⁴ are as defined above,
step f3)
substitution of mesylate-group of compound of formula VIII to an azido to form the compound of formula 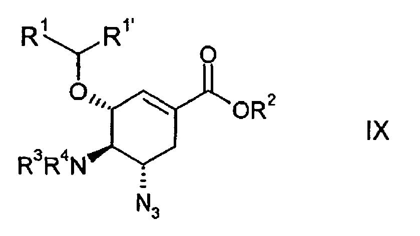 wherein R^{'}, R^{1'}, R², R³ and R⁴ are as defined above.

3. Process of claim 1, wherein step f) comprises the steps,
step f'1)
substitution of mesylate-group of compound of formula VII to form the compound of formula 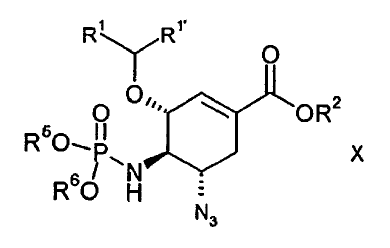 wherein R¹, R^{1'}, R², R³ and R⁴ are as defined above,
step f'2)
hydrolysis of compound of formula X to form compound of formula 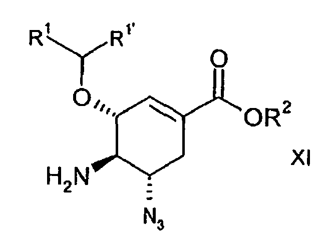 wherein R¹, R^{1'} and R² are as defined above,
step f'3)
acylation of compound of formula XI to form compound of formula 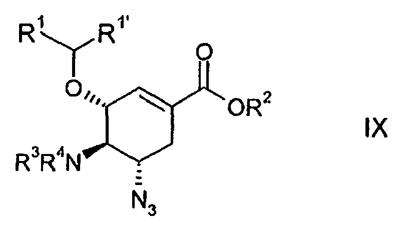 wherein R¹, R^{1'}, R², R³ and R⁴ are as defined above.

4. Process of claim 1, wherein R¹, R^{1'} and R² are ethyl, R³ is acetyl and R⁴ is hydrogen.

5. Process of claim 1, wherein R⁵ and R⁶ independently of each other are ethyl.

6. Process of claim 1, wherein the pharmaceutically acceptable acid addition salt is phosphoric acid salt.

7. Process of claim 1, wherein step a) is performed in an alcohol, preferably ethanol in the presence of a strong acid, preferably thionyl chloride at a reaction temperature range of 60 °C to 150 °C, preferably 70 °C to 100 °C.

8. Process of claim 1, wherein step b) is performed in an aprotic organic solvent, preferably ethylacetat, and methanesulfony chloride with a tertiary organic base, preferably triethylamine at reaction temperature in the range of -20°C to 50°C, preferably 0°C to 5°C.

9. Process of claim 1, wherein step c) is performed in an inert organic solvent, preferably dimethyl sulfoxide or with a water organic solvent mixture, preferably ethylacetate and with a phase transfer catalyst, such as tetrabutylammonium hydrogen sulfate and an azide reagent, preferably sodium azide at room temperature.

10. Process of claim 1, wherein step d) is performed in an organic solvent, preferably toluene in an inert gas atmosphere, trialkylphosphite, such as triethyphosphit is added at room temperature, the reaction temperature lies in the range of 50°C to 150°C, preferably 100°C to 120°C.

11. Process of claim 1, wherein step e) is performed in an alcohol, such as 3-pentanol and boron trifluoride ethyl etherate in an inert gas atmosphere at a temperature range of -20° C to 100° C, preferably 0°C to 5°C.

12. Process of claim 2, wherein step f1) is performed in an alcohol, such as ethanol and a strong acid, preferably sulfuric acid in an inert gas atmosphere, at a temperature range of 50°C to 150°C, preferably 80°C to 120°C.

13. Process of claim 2, wherein step f2) is performed by unsing acylating agent, preferably under basic conditions using acetic anhydride in ethyl acetate.at a temperature range of -20°C to 100°C.

14. Process of claim 2, wherein step f3) is performed in an inert organic solvent, preferably dimethyl sulfoxide or a solvent mixture with dimethyl sulfoxide and ethanol with an azide reagent, preferably sodium azide at 20°C to 120°C, preferably at 80°C to 100°C

15. Process of claim 3, wherein step f1) is performed in an inert organic solvent, preferably dimethyl sulfoxide or an solvent mixture with dimethyl sulfoxide and ethanol and an azide reagent, preferably sodium azide at 20°C to 120°C, preferably at 80°C to 100°C.

16. Process of claim 3, wherein step f2) is performed in an alcohol, preferably ethanol and a strong acid, preferably sulfuric acid in an inert gas atmosphere at a temperature range of 20°C to 150°C, preferably 80°C to 120°C.

17. Process of claim 3, wherein step f3) is performed under basic conditions using a mixture of acetic anhydride in ethyl acetate at temperature range of -20°C to 100°C, preferably 0 °C to 5 °C.

## Patentansprüche

1. Verfahren zur Herstellung eines 4,5-Diaminoshikimisäure-Derivats der Formel 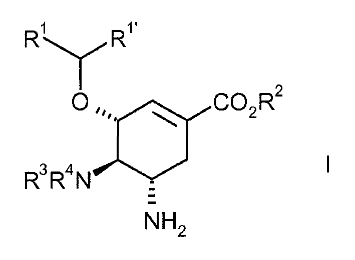 und pharmazeutisch verträglicher Additionssalze davon,
wobei
R¹, R^{1'} unabhängig voneinander H oder C₁₋₆-Alkyl sind,
R² ein C₁₋₆-Alkyl ist und
R³, R⁴ unabhängig voneinander H oder ein C₁₋₆-Alkanoyl sind, mit der Maßgabe, dass nicht beide Reste R³ und R⁴ gleich H sind,
**gekennzeichnet durch**
Schritt a)
Veresterung von Shikimisäure der Formel 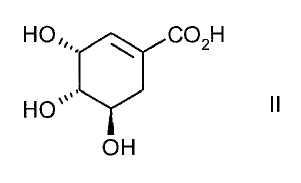 mit R²OH, um eine Verbindung der Formel 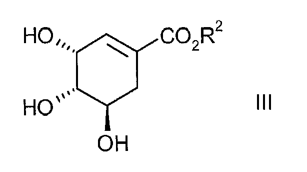 zu bilden, wobei R² wie vorstehend definiert ist,
Schritt b)
Formulierung von Trimesylat der Formel IV **durch** Umsetzen einer Verbindung der Formel III mit Methansulfonylchlorid 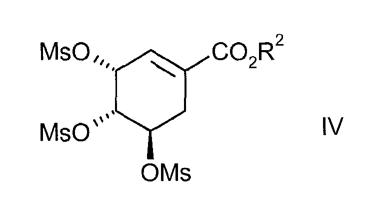 wobei R² wie vorstehend definiert ist,
Schritt c)
regioselektive S_{N}2-Substitution des 3-Mesylatrests des Trimesylats IV **durch** ein Azidreagens, um ein Azid der Formel 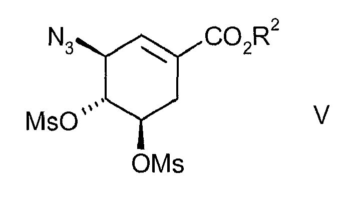 zu bilden, wobei R² wie vorstehend definiert ist,
Schritt d)
Reduktion des Azids der Formel V mit Trialkylphosphit, um ein Aziridin der Formel 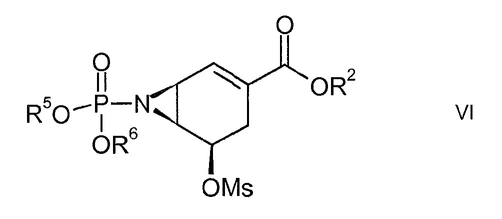 zu bilden, wobei R² wie vorstehend definiert ist, R⁵ und R⁶ unabhängig voneinander C₁₋₆-Alkyl sind,
Schritt e)
Öffnen des Aziridins der Formel VI, um eine Verbindung der Formel 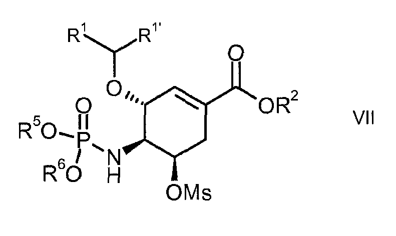 zu bilden, wobei R¹, R^{1'}, R², R⁵ und R⁶ wie vorstehend definiert sind,
Schritt f)
Umwandeln des Phosphoramids der Formel VII in eine Verbindung der Formel 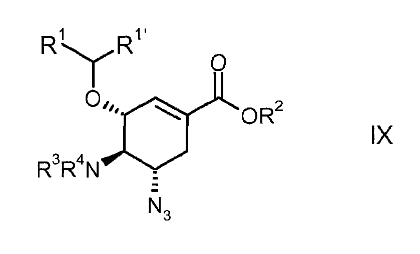 wobei R¹, R^{1'}, R², R³ und R⁴ wie vorstehend definiert sind,
Schritt g)
Reduktion des Azids der Formel IX, um eine Verbindung der Formel I zu bilden, und, wenn nötig, Bilden eines pharmazeutisch verträglichen Additionssalzes.

2. Verfahren nach Anspruch 1, wobei Schritt f) die Schritte umfasst:
Schritt f1)
Hydrolyse des Phosphoramids der Formel VII, um eine Verbindung der Formel 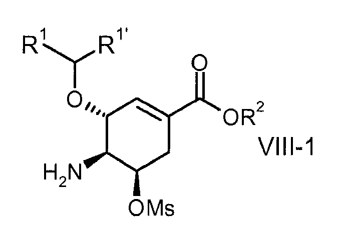 zu bilden, wobei R¹, R^{1'} und R² wie vorstehend definiert sind,
Schritt f2)
Acylierung einer Verbindung der Formel VIII-1, um eine Verbindung der Formel 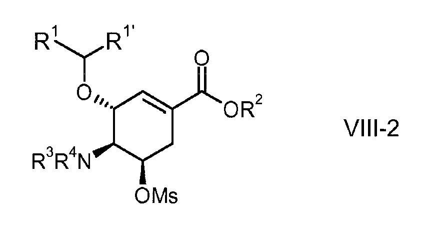 zu bilden, wobei R¹, R^{1'}, R², R³ und R⁴ wie vorstehend definiert sind,
Schritt f3)
Substitution des Mesylatrests der Verbindung der Formel VIII-2 durch einen Azidorest, um die Verbindung der Formel 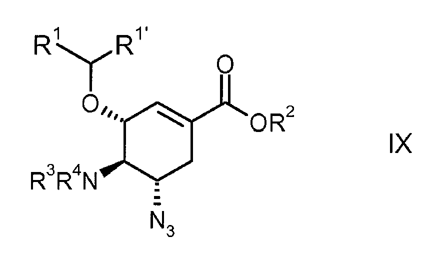 zu bilden, wobei R¹, R^{1'}, R², R³ und R⁴ wie vorstehend definiert sind.

3. Verfahren nach Anspruch 1, wobei Schritt f) die Schritte umfasst:
Schritt f1)
Substitution des Mesylatrests der Verbindung der Formel VII, um die Verbindung der Formel 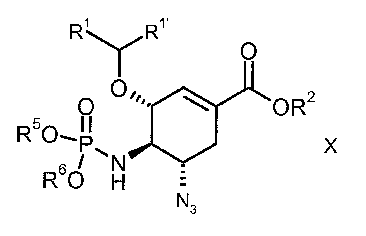 zu bilden, wobei R¹, R^{1'}, R², R³ und R⁴ wie vorstehend definiert sind,
Schritt f2)
Hydrolyse der Verbindung der Formel X, um die Verbindung der Formel 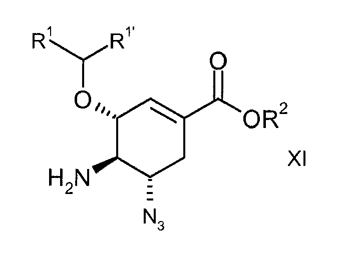 zu bilden, wobei R¹, R^{1'} und R² wie vorstehend definiert sind,
Schritt f3)
Acylierung der Verbindung der Formel XI, um eine Verbindung der Formel 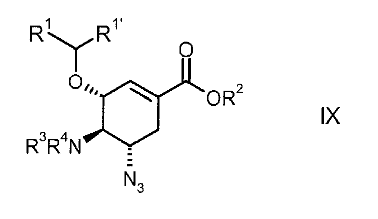 zu bilden, wobei R¹, R^{1'}, R², R³ und R⁴ wie vorstehend definiert sind.

4. Verfahren nach Anspruch 1, wobei R¹, R^{1'} und R² Ethyl sind, R³ Acetyl ist und R⁴ Wasserstoff ist.

5. Verfahren nach Anspruch 1, wobei R⁵ und R⁶ unabhängig voneinander Ethyl sind.

6. Verfahren nach Anspruch 1, wobei das pharmazeutisch verträgliche Säureadditionssalz Phosphorsäuresalz ist.

7. Verfahren nach Anspruch 1, wobei Schritt a) in einem Alkohol, bevorzugt Ethanol, in der Gegenwart einer starken Säure, bevorzugt Thionylchlorid, in einem Reaktionstemperaturbereich von 60°C bis 150°C, bevorzugt 70°C bis 100°C, durchgeführt wird.

8. Verfahren nach Anspruch 1, wobei Schritt b) in einem aprotischen organischen Lösungsmittel, bevorzugt Ethylacetat, und Methansulfonylchlorid mit einer tertiären organischen Base, bevorzugt Triethylamin, bei einer Reaktionstemperatur im Bereich von -20°C bis 50°C, bevorzugt 0°C bis 5°C, durchgeführt wird.

9. Verfahren nach Anspruch 1, wobei Schritt c) in einem inerten organischen Lösungsmittel, bevorzugt Dimethylsulfoxid, oder mit einem Gemisch aus Wasser und organischem Lösungsmittel, bevorzugt Ethylacetat, und mit einem Phasentransferkatalysator, wie Tetrabutylammoniumhydrogensulfat, und einem Azidreagens, bevorzugt Natriumazid, bei Raumtemperatur durchgeführt wird.

10. Verfahren nach Anspruch 1, wobei Schritt d) in einem organischen Lösungsmittel, bevorzugt Toluol, unter einer Inertgasatmosphäre durchgeführt wird, Trialkylphosphit, wie Triethylphosphit, bei Raumtemperatur hinzugefügt wird, die Reaktionstemperatur im Bereich von 50°C bis 150°C, bevorzugt 100°C bis 120°C, liegt.

11. Verfahren nach Anspruch 1, wobei Schritt e) in einem Alkohol, wie 3-Pentanol, und Bortrifluoridethyletherat unter einer Inertgasatmosphäre in einem Temperaturbereich von -20°C bis 100°C, bevorzugt 0°C bis 5°C, durchgeführt wird.

12. Verfahren nach Anspruch 2, wobei Schritt f1) in einem Alkohol, wie Ethanol, und einer starken Säure, bevorzugt Schwefelsäure, unter einer Inertgasatmosphäre in einem Temperaturbereich von 50°C bis 150°C, bevorzugt 80°C bis 120°C, durchgeführt wird.

13. Verfahren nach Anspruch 2, wobei Schritt f2) unter Verwendung eines Acylierungsmittels, bevorzugt unter basischen Bedingungen, unter Verwendung von Essigsäureanhydrid in Ethylacetat in einem Temperaturbereich von -20°C bis 100°C durchgeführt wird.

14. Verfahren nach Anspruch 2, wobei Schritt f3) in einem inerten organischen Lösungsmittel, bevorzugt Dimethylsulfoxid, oder einem Lösungsmittelgemisch mit Dimethylsulfoxid und Ethanol mit einem Azidreagens, bevorzugt Natriumazid, bei 20°C bis 120°C, bevorzugt bei 80°C bis 100°C, durchgeführt wird.

15. Verfahren nach Anspruch 3, wobei Schritt f1) in einem inerten organischen Lösungsmittel, bevorzugt Dimethylsulfoxid, oder einem Lösungsmittelgemisch mit Dimethylsulfoxid und Ethanol und einem Azidreagens, bevorzugt Natriumazid, bei 20°C bis 120°C, bevorzugt bei 80°C bis 100°C, durchgeführt wird.

16. Verfahren nach Anspruch 3, wobei Schritt f2) in einem Alkohol, bevorzugt Ethanol, und einer starken Säure, bevorzugt Schwefelsäure, unter einer Inertgasatmosphäre in einem Temperaturbereich von 20°C bis 150°C, bevorzugt 80°C bis 120°C, durchgeführt wird.

17. Verfahren nach Anspruch 3, wobei Schritt f3) unter basischen Bedingungen unter Verwendung eines Gemisches aus Essigsäureanhydrid in Ethylacetat in einem Temperaturbereich von -20°C bis 100°C, bevorzugt 0°C bis 5°C, durchgeführt wird.

## Revendications

1. Procédé de préparation d'un dérivé d'acide 4,5-diaminoshikimique de formule 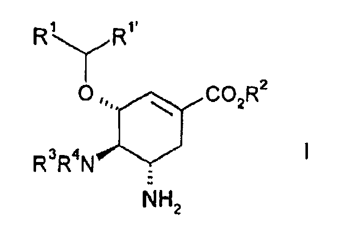 et de ses sels d'addition pharmaceutiquement acceptables,
où
R¹, R^{1'} sont, indépendamment l'un de l'autre, H ou alkyle en C₁-C₆,
R² est un alkyle en C₁-C₆ et
R³, R⁴ sont, indépendamment l'un de l'autre, H ou un alcanoyle en C₁-C₆, à condition que R³ et R⁴ ne soient pas tous les deux H,
**caractérisé en ce que**
étape a)
on estérifie l'acide shikimique de formule 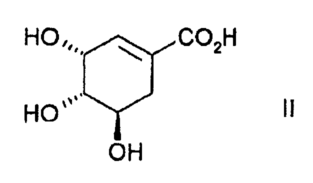 avec R²OH pour former un composé de formule 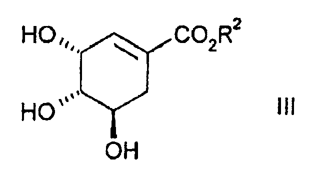 où R² est tel que défini ci-dessus;
étape b)
on forme le trimésylate de formule IV par réaction du composé de formule III avec du chlorure de méthanesulfonyle 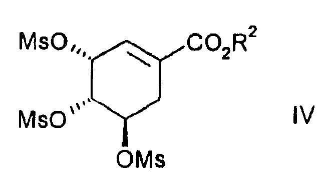 où R² est tel que défini ci-dessus;
étape c)
on effectue une substitution de type S_{N}2 régiosélective du groupe 3-mésylate du trimésylate IV par un réactif de type azide pour former un azide de formule 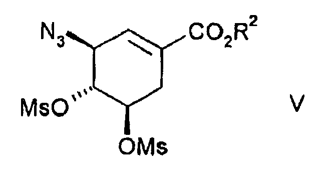 où R² est tel que défini ci-dessus;
étape d)
on réduit l'azide de formule V avec un phosphite de trialkyle pour former une aziridine de formule 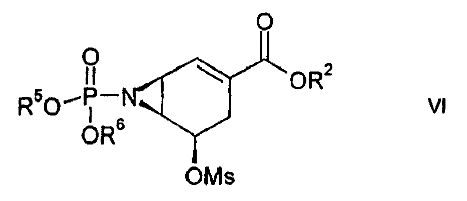 où R² est tel que défini ci-dessus, R⁵ et R⁶ sont, indépendamment l'un de l'autre, un alkyle en C₁-C₆;
étape e)
on ouvre l'aziridine de formule VI pour former le composé de formule 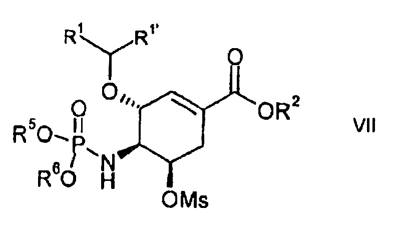 où R¹, R^{1'}, R², R⁵ et R⁶ sont tels que définis ci-dessus;
étape f)
on transforme le phosphoramide de formule VII en le composé de formule 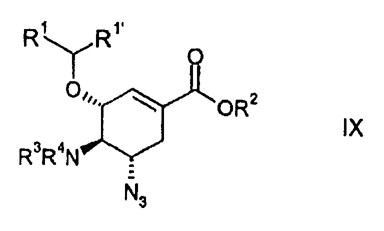 où R¹, R^{1'}, R², R³ et R⁴ sont tels que définis ci-dessus;
étape g)
on réduit l'azide de formule IX pour former un composé de formule I et, si nécessaire, on forme un sel d'addition pharmaceutiquement acceptable.

2. Procédé selon la revendication 1, dans lequel l'étape f) comprend les étapes:
étape f1)
hydrolyse du phosphoramide de formule VII pour former le composé de formule 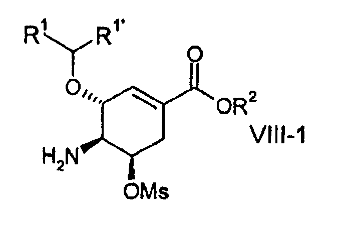 où R¹, R^{1'} et R² sont tels que définis ci-dessus;
étape f2)
acylation du composé de formule VIII-1 pour former le composé de formule 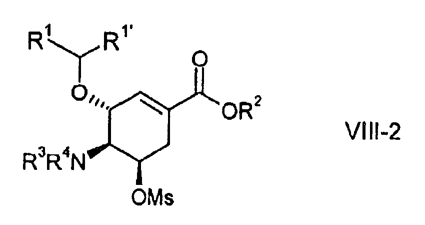 où R¹, R^{1'}, R², R³ et R⁴ sont tels que définis ci-dessus;
étape f3)
substitution du groupe mésylate du composé de formule VIII en un azido pour former le composé de formule 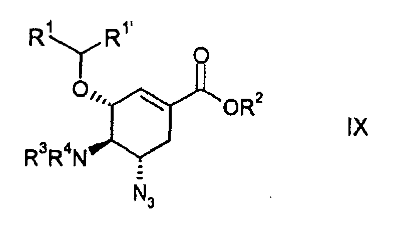 où R¹, R^{1'}, R², R³ et R⁴ sont tels que définis ci-dessus.

3. Procédé selon la revendication 1, dans lequel l'étape f) comprend les étapes:
étape f1)
substitution du groupe mésylate du composé de formule VII pour former le composé de formule 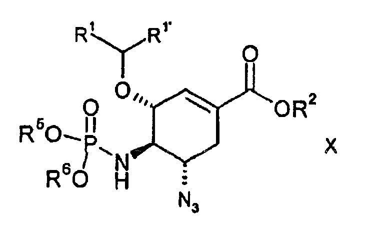 où R¹, R¹, R², R³ et R⁴ sont tels que définis ci-dessus;
étape f2)
hydrolyse du composé de formule X pour former le composé de formule 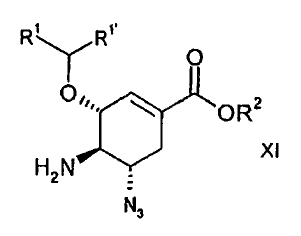 où R¹, R^{1'} et R² sont tels que définis ci-dessus;
étape f3)
acylation du composé de formule XI pour former le composé de formule 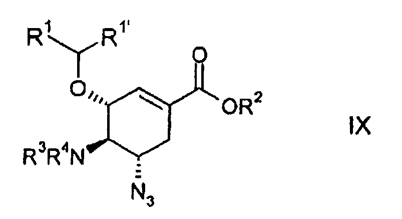 où R¹, R^{1'}, R², R³ et R⁴ sont tels que définis ci-dessus.

4. Procédé selon la revendication 1, dans lequel R¹, R^{1'} et R² sont des groupes éthyle, R³ est un acétyle et R⁴ est un hydrogène.

5. Procédé selon la revendication 1, dans lequel R⁵ et R⁶ sont, indépendamment l'un de l'autre, un éthyle.

6. Procédé selon la revendication 1, dans lequel le sel d'addition d'acide pharmaceutiquement acceptable est un sel d'acide phosphorique.

7. Procédé selon la revendication 1, dans lequel l'étape a) s'effectue dans un alcool, de préférence l'éthanol, en présence d'un acide fort, de préférence le chlorure de thionyle, à une température de réaction se situant dans le domaine de 60°C à 150°C, de préférence de 70°C à 100°C.

8. Procédé selon la revendication 1, dans lequel l'étape b) s'effectue dans un solvant organique aprotique, de préférence l'acétate d'éthyle, et du chlorure de méthanesulfonyle avec une base organique tertiaire, de préférence la triéthylamine, à une température de réaction se situant dans le domaine de -20°C à 50°C, de préférence de 0°C à 5°C.

9. Procédé selon la revendication 1, dans lequel l'étape c) s'effectue dans un solvant organique inerte, de préférence le diméthylsulfoxyde, ou avec un mélange d'eau et de solvant organique, de préférence l'acétate d'éthyle, et avec un catalyseur de transfert de phase comme l'hydrogénosulfate de tétrabutylammonium et et un réactif de type azide, de préférence l'azoture de sodium, à la température ambiante.

10. Procédé selon la revendication 1, dans lequel l'étape d) s'effectue dans un solvant organique, de préférence le toluène, dans une atmosphère de gaz inerte, on ajoute un phosphite de trialkyle comme le phosphite de triéthyle à la température ambiante, la température de réaction se situe dans le domaine de 50°C à 150°C, de préférence de 100°C à 120°C.

11. Procédé selon la revendication 1, dans lequel l'étape e) s'effectue dans un alcool comme le 3-pentanol et du trifluorure de bore-éthérate d'éthyle dans une atmosphère de gaz inerte à une température se situant dans le domaine de -20°C à 100°C, de préférence de 0°C à 5°C.

12. Procédé selon la revendication 2, dans lequel l'étape f1) s'effectue dans un alcool comme l'éthanol et un acide fort, de préférence l'acide sulfurique, dans une atmosphère de gaz inerte, à une température se situant dans le domaine de 50°C à 150°C, de préférence de 80°C à 120°C.

13. Procédé selon la revendication 2, dans lequel l'étape f2) s'effectue à l'aide d'un agent d'acylation, de préférence dans des conditions basiques, avec de l'anhydride acétique dans de l'acétate d'éthyle à une température se situant dans le domaine de -20°C à 100°C.

14. Procédé selon la revendication 2, dans lequel l'étape f3) s'effectue dans un solvant organique inerte, de préférence le diméthylsulfoxyde ou un mélange de solvants avec du diméthylsulfoxyde et de l'éthanol, avec un réactif de type azide, de préférence l'azoture de sodium, à une température de 20°C à 120°C, de préférence de 80°C à 100°C.

15. Procédé selon la revendication 3, dans lequel l'étape f1) s'effectue dans un solvant organique inerte, de préférence le diméthylsulfoxyde ou un mélange de solvants avec du diméthylsulfoxyde et de l'éthanol, et un réactif de type azide, de préférence l'azoture de sodium, à une température de 20°C à 120°C, de préférence de 80°C à 100°C.

16. Procédé selon la revendication 3, dans lequel l'étape f2) s'effectue dans un alcool, de préférence l'éthanol, et un acide fort, de préférence l'acide sulfurique, dans une atmosphère de gaz inerte, à une température se situant dans le domaine de 20°C à 150°C, de préférence de 80°C à 120°C.

17. Procédé selon la revendication 3, dans lequel l'étape f3) s'effectue dans des conditions basiques à l'aide d'un mélange d'anhydride acétique dans de l'acétate d'éthyle, à une température se situant dans le domaine de -20°C à 100°C, de préférence de 0°C à 5°C.
